# EUROPEAN PATENT SPECIFICATION

(11) **EP 3 626 074 B1**
(45) Date of publication and mention of the grant of the patent: **29.03.2023**
(21) Application number: 18801422.9
(22) Date of filing: 18.05.2018
(51) Int. Cl.: A01K 67/033, C12N 15/54, G01N 33/15, G01N 33/50, C12N 9/10, C12N 15/09, C12N 15/00

(54) **A TRANSGENIC INVERTEBRATE MODEL FOR ALZHEIMER'S DISEASE AND USE THEREOF**
TRANSGENESMODELL DER ALZHEIMER-KRANKHEIT BEI WIRBELLOSEN UND VERWENDUNG DAVON
MODÈLE INVERTÉBRÉ TRANSGÉNIQUE DE LA MALADIE D'ALZHEIMER ET SON UTILISATION

(30) Priority: 19.05.2017 JP 2017099855
(43) Date of publication of application: 25.03.2020
(73) Proprietor: National Center for Geriatrics and Gerontology, Aichi 474-8511 (JP)
(72) Inventor: YAMASAKI, Yasutoyo, Obu-shi Aichi 474-8511 (JP); YANAGISAWA, Katsuhiko, Obu-shi Aichi 474-8511 (JP); TSUDA, Leo, Obu-shi Aichi 474-8511 (JP)
(74) Representative: HGF
(86) International application number: PCT/JP2018/019275
(87) International publication number: WO 2018/212319

(56) References cited:
- WO-A1-2005/041650
- TOSHIO ARIGA ET AL: "Thematic Review Series: Sphingolipids. Role of ganglioside metabolism in the pathogenesis of Alzheimer's disease?a review", JOURNAL OF LIPID RESEARCH, AMERICAN SOCIETY FOR BIOCHEMISTRY AND MOLECULAR BIOLOGY, INC, US, vol. 49, no. 6, June 2008 (2008-06), pages 1157-1175, XP008152660, ISSN: 0022-2275, DOI: 10.1194/JLR.R800007-JLR200 [retrieved on 2008-03-11]
- C. A. STERN ET AL: "Molecular identification, tissue distribution and subcellular localization of mST3GalV/GM3 synthase", GLYCOBIOLOGY, vol. 10, no. 4, April 2000 (2000-04), pages 365-374, XP055764214, US ISSN: 0959-6658, DOI: 10.1093/glycob/10.4.365
- KANAE IIJIMA-ANDO ET AL: "Transgenic Drosophila models of Alzheimer's disease and tauopathies", BRAIN STRUCTURE AND FUNCTION, SPRINGER, BERLIN, DE, vol. 214, no. 2-3, 5 December 2009 (2009-12-05), pages 245-262, XP019803161, ISSN: 1863-2661
- DEBBY VAN DAM ET AL: "Animal models in the drug discovery pipeline for Alzheimer's disease : Animal models for AD", BRITISH JOURNAL OF PHARMACOLOGY, vol. 164, no. 4, October 2011 (2011-10), pages 1285-1300, XP055763808, UK ISSN: 0007-1188, DOI: 10.1111/j.1476-5381.2011.01299.x
- CROWTHER, D. C. et al.: "Intraneuronal A , non-amyloid aggregates and neurodegeneration in a Drosophila model of Alzheimer s disease", Neuroscience, vol. 132, no. 1, 2005, pages 123-135, XP004789351,
- CHANG, K. H. et al.: "Expression of recombinant cyclooxygenase 1 in Drosophila melanogaster S2 cells transformed with human beta 1, 4-galactosyltransferase and Gal beta 1, 4-GlcNAc a 2, 6-sialyltransferase", Biotechnology Letters, vol. 29, no. 12, 2007, pages 1803-1809, XP019548729,
- SUZUKI et al: "Studying human neurodegenerative diseases using Drosophila melanogaster", SEITAI NO KAGAKU, vol. 67, no. 6, 5 December 2016 (2016-12-05), pages 589-595, XP009518043,
- Shinobu Kitazume: "Glycosyltransferasse as subtrate for Alzheimer´s disease beta-secretase", Protein , nucleic acid and enzyme, vol. 49, no. 15, 2004, pages 2468-2472, XP009517997, ISSN: 0039-9450
- TSUDA et al: "178: Development of therapeutic drug for Alzheimer´s disease by screening individual level", Dementia Japan, vol. 29, no. 3, 15 September 2015 (2015-09-15), page 163, XP009517999, ISSN: 1342-646X
- TSUDA et al: "Creation of quantitative analysis model for neurodegeneration accompanying onset of Alzheimer´s disease", Dementia Japan, vol. 30, no. 4, 15 October 2016 (2016-10-15), page 178, XP009518000, ISSN: 1342-646X

## Description

### [Technical Field]

The present invention relates to a transgenic Drosophila model for Alzheimer's disease and to a method for screening therapeutic agents for Alzheimer's disease using the same.

### [Background Art]

Currently, the population is aging at a faster rate than ever in Japan, and consequently, the number of patients with dementia, predominantly Alzheimer's disease, has been increasing. Since caretaking of dementia patients imposes a large economic burden, establishment of an effective therapy is urgently required. At present, for treating Alzheimer-type dementia, symptoms are treated using, e.g., a cholinesterase inhibitor and an NMDA inhibitor, is employed for temporality improving the symptoms of dementia; however, the effect of the symptomatic treatment is extremely limited. In these circumstances, establishment of a causal therapy for suppressing onset and progression of Alzheimer's disease is strongly desired.

The establishment of a causal therapy of Alzheimer's disease requires elucidation of the pathogenic mechanism of Alzheimer's disease. The following three features are known as pathological features commonly seen in patients with Alzheimer's disease: (1) shrinkage of the brain, (2) formation of plaque-like accumulation of amyloid (senile plaque) and (3) formation of fibrous mass within neurons (neurofibrillary tangles (NFT)). Senile plaque is known to be formed by aggregation/accumulation of amyloid β (Aβ). The amyloid hypothesis that Alzheimer's disease is developed first from aggregation of Aβ is proposed and deemed as a particularly popular hypothesis. It has been found that the aggregation of Aβ in the brain starts with binding of Aβ to GM1 ganglioside, constituting the cell membrane of neurons, to form a complex (GM1-bound Aβ (GAβ)) (Non-Patent Document 1). Many research reports supporting the hypothesis that aggregation of Aβ is facilitated from a "seed" of GAβ have been obtained (Non-Patent Documents 2 and 3).

The use of model animals is extremely useful for further elucidating mechanism how to develop Alzheimer's disease and for developing a therapeutic agent for a causal therapy for Alzheimer's disease. Up to the present, many mouse models for Alzheimer's disease have been prepared and used in research. However, it has been difficult to analyze the interaction between Aβ and gangliosides, involved in pathology of Alzheimer's disease, because endogenous gangliosides are present in large amounts in mammals, acting as a disincentive. In addition, there are problems in that testing a large number of candidate compounds as therapeutic agents by using mice is time consuming and costly because a considerably large number of mice are required.

In contrast, endogenous expression of a ganglioside is not detected in invertebrates such as insects and nematodes. In addition, compared to mammals, invertebrates have a short life cycle and can be easily raised at low cost. These features are advantageous for large-scale screening of therapeutic agent candidate compounds. However, successful preparations of invertebrates producing a ganglioside have not yet been reported.

### [Citation List]

### [Non-Patent Documents]

[Non-Patent Document 1] Yanagisawa, K. et al., Nat. Med., Vol. 1, pp. 1062-1066 (1995)
[Non-Patent Document 2] Hayashi, H. et al., J. Nuerosci., Vol. 24, pp. 4894-4902 (2004)
[Non-Patent Document 3] Yanagisawa K., Glycoconj. J., Vol. 32, pp. 87-91 (2015)

### [Summary of Invention]

### [Technical Problem]

An object of the present invention is to solve problems in the prior art and to provide an invertebrate mimicking the aggregation of Aβ in the physiological conditions of the human brain.

### [Solution to Problem]

As a result of diligent studies, the present inventors have succeeded in providing a transgenic *Drosophila* producing a GM3 ganglioside. The present inventors have found that aggregation of Aβ in the physiological conditions of the human brain can be mimicked by co-expressing Dutch amyloid β mutant in the transgenic *Drosophila* producing a GM3 ganglioside.

More specifically, according to an embodiment, the present invention is to provide a transgenic *Drosophila,* wherein genes encoding mammalian β-1,4-galactose transferase and α-2,3-sialyltransferase are introduced, and GM3 ganglioside is expressed.

Preferably, the transgenic *Drosophila* is a transgenic *Drosophila* in which a gene encoding an enzyme involved in synthesis of sialic acid is further introduced.

Preferably, the transgenic *Drosophila* is a transgenic *Drosophila,* in which a gene encoding amyloid β is further introduced, and the GM3 ganglioside and amyloid β are co-expressed, wherein the amyloid β is a Dutch amyloid β mutant.

According to an embodiment, the present invention is to provide a method for screening a therapeutic agent for Alzheimer's disease, comprising the steps of: (1) providing the transgenic *Drosophila* co-expressing the GM3 ganglioside and Dutch amyloid β mutant according to the invention; (2) administering a candidate compound to the transgenic *Drosophila;* and (3) analyzing the transgenic *Drosophila* after administration of the candidate compound in the step (2).

### [Advantageous Effects of Invention]

In the transgenic *Drosophila* according to the present invention, since an endogenous ganglioside is not present, aggregation of Aβ in the physiological condition of the human brain can be reproduced by allowing the same-level of ganglioside as in the human brain and Aβ to express. If the transgenic *Drosophila* according to the present invention is used for screening a therapeutic agent for Alzheimer's disease, a large number of candidate compounds can be quickly screened at low cost with high throughput.

### [Brief Description of Drawings]

[Fig. 1] Fig. 1 is a diagram showing a synthesis pathway for GM3 ganglioside in a transgenic *Drosophila.*
[Fig. 2] Fig. 2 shows photographs representing the expression of GALT6, SAT1 and GNE in the neural tissue of a transgenic *Drosophila* confirmed by immunostaining.
[Fig. 3] Fig. 3 shows (a) a mass spectrum of lipids extracted from the cerebral neural tissue of a transgenic *Drosophila* expressing GALT6 and SAT1 (Neu5Ac feeding (-)), and (b) a mass spectrum of lipids extracted from cerebral neural tissue of a transgenic *Drosophila* expressing GALT6 and SAT1 (Neu5Ac feeding (+)).
[Fig. 4] Fig. 4 is Western blot showing the detection results of Aβ aggregation in the cerebral neural tissue of a transgenic *Drosophila* (Neu5Ac feeding (+)) expressing GALT6, SAT1 and Dut40.
[Fig. 5] Fig. 5 is a graph quantitatively showing the results of Fig. 4.
[Fig. 6] Fig. 6 is Western blot showing the detection of Aβ aggregation in the cerebral neural tissue of a transgenic *Drosophila* (Neu5Ac feeding (-) or (+)) expressing only Dut40.
[Fig. 7] Fig. 7 is a diagram showing the principle of the method for screening a therapeutic agent for Alzheimer's disease using a transgenic Drosophila, according to an embodiment of the present invention.

### [Description]

Hereinafter, the present disclosure will be described in detail.

Described herein is a transgenic invertebrate expressing a ganglioside, in which genes encoding enzymes involved in production of a ganglioside are introduced.

The "invertebrate" described may be any invertebrate as long as it does not substantially express an endogenous ganglioside, i.e., an invertebrate of lower rank than an echinoderm. The phrase "not substantially express an endogenous ganglioside" means that an endogenous ganglioside cannot be detected by a detection means ordinarily used (for example, immunochemical method). Examples of an invertebrate which does not substantially express an endogenous ganglioside include arthropods such as insects, and nemathelminths such as nematodes, annelids and mollusks. The invertebrate may be an insect or a nematode (*Caenorhabditis elegans*)*.* Examples of an insect include an insect in the *Diptera* such as *Drosophila,* an insect of the *Lepidoptera* such as a silkworm, an insect of the *Hymenoptera* such as a bee and an insect of the *Coleoptera* such as a flour beetle. The insect may be a fly such as *Drosophila,* and particularly preferably, *Drosophila melanogaster.* According to the invention, the transgenic invertebrate is *Drosophila.*

The "ganglioside" described refers to a glycosphingolipid, which is a glycolipid obtained by binding a sugar to a ceramide and containing at least one sialic acid in the sugar chain moiety. Gangliosides are classified into, e.g., a ganglio series, a lacto series and globo series according to the structure of the sugar chain moiety, and further classified based on the number of sialic acids contained in the sugar chain moiety. Examples of the ganglio-series ganglioside containing a single sialic acid include GM1a, GM1b, GM2 and GM3. Examples of a ganglioside-series ganglioside containing two sialic acids include GD1a, GD1b, GD2 and GD3. Examples of a ganglioside-series ganglioside containing three sialic acids include GT1a, GT1b, GT1c, GT2 and GT3. Examples of a ganglioside-series ganglioside containing four sialic acids include GQ1b and GQ1c. Examples of a ganglioside-series ganglioside containing five sialic acids include GP1c. The ganglioside may be a monosialoganglioseries ganglioside such as GM1a, GM1b, GM2 and GM3. According to the invention the ganglioside is GM3.

Examples of the "enzyme involved in production of a ganglioside" may include glycosyltransferase and glycoside hydrolase. Examples of the glycosyltransferase include galactosyltransferase, sialyltransferase, fucosyltransferase and N-acetylgalactosamine transferase. Examples of the glycoside hydrolase include galactosidase, glucosidase and N-acetylglucosaminidase. As the biosynthesis pathway of a ganglioside has been elucidated and suitable enzymes can be selected in accordance with the ganglioside to be desirably expressed. For example, GM3 can be produced by expressing β-1,4-galactose transferase and α-2,3-sialyltransferase. For example, GM2 can be produced by expressing β-1,4-N-acetylgalactosamine transferase. For example, GM1a can be produced by expressing β-1,3-galactose transferase 2. In the transgenic *Drosophila* according to the invention, genes encoding mammalian β-1,4-galactose transferase and α-2,3-sialyltransferase are introduced.

Enzymes involved in production of a ganglioside may be derived from any animal species, preferably a mammal such as a mouse, a rat, a rabbit, a dog, a non-human primate and a human, and particularly preferably, a human. Genes encoding enzymes involved in production of a ganglioside have already been cloned and the information of the nucleotide sequences is available from a predetermined database. For example, for human β-1,4-galactose transferase, accession number BC069620.1 in the GenBank is available. For human α 2,3-sialyltransferase, accession numbers NM_003896 and NM_001042437 in the GenBank are available. For human β 1,4-N-acetylgalactosamine transferase, accession numbers NM_153446, NM_001159387.1 and NM_001159388.1 in the GenBank are available. For human β 1,3-galactose transferase 2, accession number AF288390 in the GenBank is available.

Examples of the enzymes involved in production of a ganglioside may include a protein consisting of an amino acid sequence having an identity of 80% or more, preferably 90% or more, more preferably about 95% or more with the above amino acid sequences registered in the database as long as it maintains enzymatic activity. The amino acid sequence identity can be determined using sequence analysis software, or using a program commonly used in the present technical field (FASTA, BLAST, etc.). Also, the enzyme involved in production of a ganglioside may include a protein consisting of an amino acid sequence registered in the database having a substitution, deletion, insertion and/or addition of one to several amino acids as long as it maintains the enzymatic activity. The "one to several" herein refers to, for example "1 to 30", preferably "1 to 10" and particularly preferably, "1 to 5".

A gene encoding an enzyme involved in production of a ganglioside can be introduced into an invertebrate by a method well-known in the art; for example, by subcloning the sequence of the gene desired to be introduced into a vector known in the art, e.g., a plasmid, a virus or a transposon, together with a transcription regulatory sequence such as a promoter, and introducing the vector into a target invertebrate. Particularly, in the case of an insect, a genetic recombination technique using a transposon is sufficiently established. For example, if *Drosophila* is used, GAL4-UAS expression system sufficiently established in the art can be used. More specifically, a transgenic *Drosophila* expressing a ganglioside can be prepared by crossing a *Drosophila* strain expressing a transcription factor GAL4, with a *Drosophila* strain, in which a vector having a gene encoding an enzyme involved in production of a ganglioside is introduced in the downstream of UAS (a target sequence of GAL4).

The *Drosophila* strain having a gene encoding an enzyme involved in production of a ganglioside downstream of UAS can be prepared by introducing a vector for transformation, such as pUAST, in which a gene encoding a desired enzyme is integrated, into an embryo of *Drosophila.* An existing *Drosophila* strain expressing GAL4 may be available. Alternatively, the *Drosophila* strain expressing GAL4 may be prepared by introducing a vector comprising a GAL4 gene in the downstream of a promoter/enhancer in accordance with a method known in the art. Preferably, a neuron-specific promoter/enhancer can be used. Although it is not particularly limited, e.g., elav, Appl and R57C10 can be used, and preferably elav can be used. When an existing strain is used as a *Drosophila* strain expressing GAL4 specifically to neurons, for example, elav^{C155}-gal4 strain (Lin, D. M. et al., Neuron, 13: 507-523 (1994)) which is a strain expressing GAL4 specifically to neurons; and mb247-gal4 strain (Schulz, RA. et al., Oncogene, 12: 1827-1831 (1996)) expressing GAL4 specifically to neurons of the brain mushroom body, can be used.

The *Drosophila* strains mentioned above can be available from, for example, domestic or international public stock centers such as *Drosophila* Genetic Resource Center of the Kyoto Institute of Technology (http://www.dgrc.kit.ac.jp/) and the Bloomington *Drosophila* Stock Center (http://flystocks.bio.indiana.edu/).

In order for a transgenic invertebrate described herein to sufficiently and successfully produce a ganglioside, it is preferable to sufficiently supply a sialic acid donor substrate for supplying sialic acid, which is a constitution component of a ganglioside. The sialic acid donor substrate may be supplied by adding it to feed or by further introducing a gene encoding an enzyme involved in synthesis of a sialic acid donor substrate to the transgenic invertebrate so as to biologically synthesize the sialic acid donor substrate. More specifically, in a transgenic invertebrate, a gene encoding an enzyme involved in synthesis a sialic acid donor substrate may be introduced in addition to a gene encoding an enzyme involved in production of a ganglioside. Examples of the gene encoding an enzyme involved in synthesis of a sialic acid donor substrate may include a gene (GenBank Accession Number NG_008246. 1) encoding human UDP-N-acetylglucosamine 2-epimerase/N-acetylmannosamine kinase. In the transgenic Drosophila of the invention, a gene encoding an enzyme involved in synthesis of sialic acid may be further introduced.

In a transgenic invertebrate described herein, in one instance a gene encoding amyloid β is further introduced, and the ganglioside and the amyloid β are co-expressed. Amyloid β (hereinafter referred to as "Aβ") refers to a peptide produced by cleaving an amyloid precursor protein fragment (APP) with β-secretase and γ-secretase. Aβ40 and Aβ42 different in number of amino acids, which are produced by cleaving APP at different points, are included.

Examples of "APP" may include not only human APP isoforms consisting of amino acid sequences registered in the RefSeq database of the NCBI (http://www.ncbi.nlm.nih.gov/RefSeq/) under RefSeq Accession Numbers NP_001129601.1, NP_001129602.1, NP_958817.1, NP_001191231.1, NP_958816.1, NP_001191230.1, NP_000475.1, NP_001129488.1 and NP_001129603.1, but also an isoform containing a mutation known in the art and found in familial Alzheimer's disease.

Examples of "APP" may include a protein consisting of an amino acid sequence having an identity of 80% or more, preferably 90% or more, more preferably about 95% or more with the amino acid sequence as mentioned above, as long as it can be cleaved with β-secretase and γ-secretase to produce Aβ. The amino acid sequence identity can be determined using sequence analysis software, or using a program commonly used in the present technical field (FASTA, BLAST, etc.).

Examples of "APP" may include a protein consisting of an amino acid sequence as mentioned above having a substitution, deletion, insertion and/or addition of one to several amino acids as long as it is cleaved with β-secretase and γ-secretase to produce Aβ. The "one to several" herein refers to, for example "1 to 30", preferably "1 to 10" and particularly preferably, "1 to 5".

More specifically, examples of Aβ may include, but are not limited to, a wild type, Aβ40 or Aβ42 such as Dutch mutant (E22Q), Arctic mutant (E22G), Italian mutant (E22K), Flemish mutant (A21G) and Iowa mutant (D23N). Aβ may be for example Dutch mutant, Aβ40 or Aβ42.

In a transgenic Drosophila according to the invention wherein a gene encoding amyloid β is further introduced, and the GM3 ganglioside, and amyloid β are co-expressed, the amyloid β is a Dutch amyloid β mutant.

The transgenic invertebrate described herein can be used as a model animal for analyzing various physiological functions such as a neural function in which ganglioside is involved. Also, a transgenic invertebrate described herein can reproduce the aggregation of Aβ under the physiological conditions of the human brain, when Aβ is co-expressed therein at a level as low as in the human brain. Thus, the transgenic invertebrate is useful as a model animal for Alzheimer's disease.

Also described herein is a method for screening a therapeutic agent for Alzheimer's disease, including the steps of: (1) providing a transgenic invertebrate co-expressing a ganglioside as described above and amyloid β; (2) administering a candidate compound to the transgenic invertebrate; and (3) analyzing the transgenic invertebrate after administration of the candidate compound in the step (2). The screening method makes it possible to obtain a compound inhibiting interaction between Aβ and a ganglioside as an Aβ aggregation inhibitor for use in definitive treatment or prevention for Alzheimer's disease.

The terms "invertebrate", "ganglioside" and "amyloid β (Aβ)" used in the described method are the same as those defined above for the transgenic invertebrate.

In the screening method according to the invention, the transgenic invertebrate is a transgenic *Drosophila* according to the invention wherein a gene encoding amyloid β is further introduced, and the GM3 ganglioside, and amyloid β are co-expressed, wherein the amyloid β is a Dutch amyloid β mutant.

The screening method described employs a transgenic invertebrate co-expressing a ganglioside and Aβ. In the method, the transgenic invertebrate co-expressing a ganglioside and Aβ can be prepared in the same manner as described above.

Then, a candidate compound is administered to the transgenic invertebrate co-expressing a ganglioside and Aβ. Examples of the candidate compound may include synthetic compounds, peptide compounds, nucleic acids and antibodies, and these candidate compounds may be novel compounds or compounds known in the art. For administering a candidate compound to a transgenic invertebrate, the candidate compound may be added to feed in various concentrations and allowed to eat. The concentration of the candidate compound to be added to the feed may vary depending on the type of compound. The concentration can be appropriately selected from, for example, the range of 0.1 to 100 mM. A candidate compound can be administered over the period from, e.g., 7 to 30 days.

Then, the transgenic invertebrate after administration of the candidate compound is analyzed so as to evaluate the Aβ aggregation inhibition effect of the candidate compound. Accumulation of aggregated Aβ can be detected and quantified by analyzing a brain tissue biochemically or pathologically in accordance with a procedure known in the art, e.g., by using an anti-Aβ antibody. The degree of accumulation of aggregated Aβ in the brain tissue can be evaluated by determining motor function and learning and memory abilities because the transgenic invertebrate deteriorates in motor function and learning and memory abilities in correlation to accumulation of aggregated Aβ,. The methods for determining motor function and learning and memory abilities have already been sufficiently established, and an appropriate method can be selected from those known in the art. For example, when *Drosophila* is used as an invertebrate, motor function can be determined by a climbing assay, sufficiently established in the art, based on evaluation of negative geotaxis. The learning and memory abilities can be determined by the odor-aversion learning test using odor and electric shock in combination.

Whether the accumulation of aggregated Aβ in the transgenic invertebrate received a candidate compound is decreased can be determined by comparing to the results of concurrent analysis of a transgenic invertebrate not administered the candidate compound, or by comparing to the results of past analysis of a transgenic invertebrate not administered the candidate compound.

In the screening method, when the accumulation amount of aggregated Aβ is significantly decreased by administration of a candidate compound, compared to the case in transgenic invertebrate not administered the candidate compound, the candidate compound can be evaluated to be a promising Aβ aggregation inhibitor which is useful for definite treatment or prevention for Alzheimer's disease. In contrast, when the accumulation amount of aggregated Aβ did not change or increased by administration of a candidate compound, compared to the case of a transgenic invertebrate to which the candidate compound not administered, the candidate compound cannot be evaluated as a promising Aβ aggregation inhibitor which is useful for definite treatment or prevention for Alzheimer's disease.

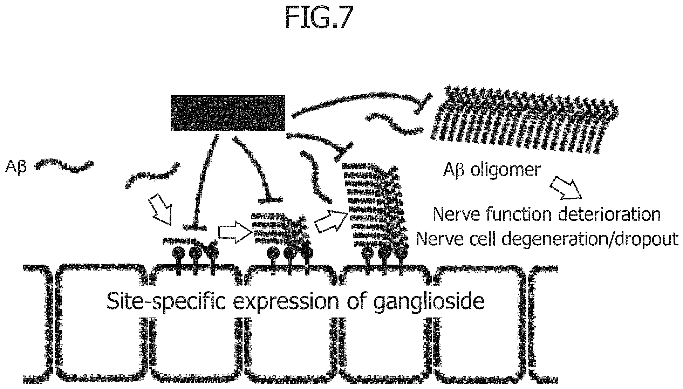

The method is schematically shown in Fig. 7. Since no endogenous ganglioside is present in an invertebrate, a ganglioside can be produced specifically and exclusively at a desired site (for example, neural tissue). As a result, accumulation of aggregated Aβ, which is expressed at a level as low as in the human brain, can be detected. Thus, the method can be used for evaluating the inhibitory effects on Aβ aggregation of a candidate compound in the similar physiological conditions to those in the human brain, and is therefore useful for screening a therapeutic agent for Alzheimer's disease.

### [Examples]

The present invention will now be further described with Examples, but is not limited to the following Examples. Note that, these examples should not be construed as limiting the present invention.

### 1. Preparation of transgenic Drosophila and breeding thereof

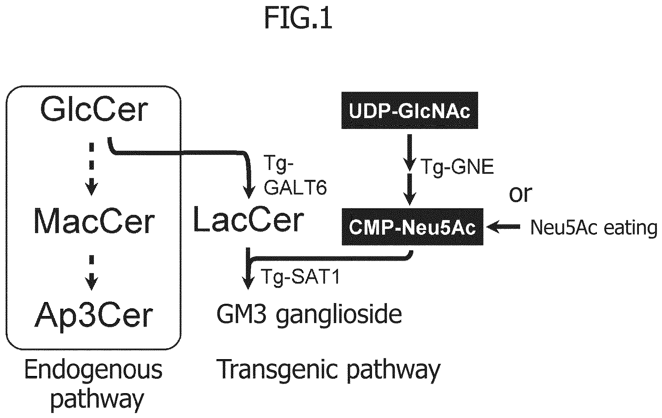

Transgenic *Drosophila,* which expresses enzymes involved in the synthesis pathway for GM3 ganglioside in mammals, i.e., β-1,4-galactose transferase (official abbreviation: B4GALT6, hereinafter referred to as "GALT6"), α-2,3-sialyltransferase (official abbreviation: ST3GAL5, hereinafter referred to as "SAT1"), and UDP-N-acetylglucosamine 2-epimerase/N-acetylmannosamine kinase (official abbreviation: GNE), was prepared in the following procedure. Fig. 1 shows the synthesis pathway for GM3 ganglioside in the transgenic *Drosophila* prepared in the Example. In the transgenic *Drosophila,* the introduction of a transgene GALT6 (Tg-GALT6) makes possible the synthesis of lactosylceramide (LacCer) from glucosylceramide (GlcCer), and the introduction of a transgene SAT1 (Tg-SAT1) makes possible the synthesis of GM3 ganglioside from LacCer. In addition, the introduction of a transgene GNE (Tg-GNE) makes possible the synthesis of CMP-Neu5Ac, a sialic acid donor substrate for synthesizing GM3 ganglioside.

The coding sequences of human GALT6, human SAT1, and human GNE were obtained by PCR amplification using a human brain cDNA library (code number 9503, Takara Bio Inc.) as templates and the following primer sets.

### (i) GALT6

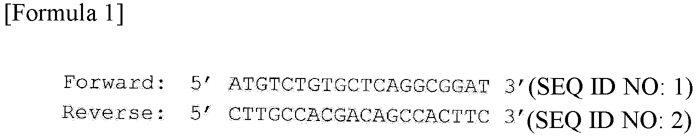

### (ii) SAT1

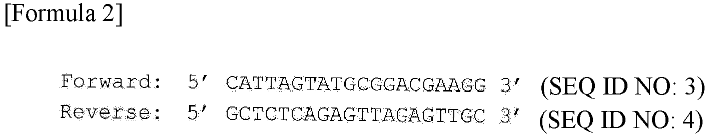

### (iii) GNE

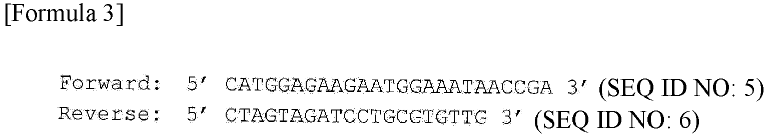

PCR products were each cloned in pCR-Blunt-II TOPO vector (Thermo Fisher Scientific) and subsequently subcloned at an EcoRI site of pUAST vector (Brand and Perrimon, Development, 1993). The pUAST vector was injected into the embryo of *Drosophila,* yw-strain. The adult *Drosophila* obtained was further crossed with *Drosophila,* w¹¹¹⁸-strain. In this manner, transgenic *Drosophila* strains UAS-GALT6, UAS-SAT1 and UAS-GNE were obtained.

A transgenic *Drosophila* expressing Dutch mutant (E22Q) Aβ40 (hereinafter referred to as "Dut40") was prepared in the following procedure. The coding sequence of human Aβ was prepared by amplifying cDNA of HeLa cells by PCR and the peptide sequence of a secretory signal of rat proenkephalin A (Finelli et al., Mol. Cell. Neurosci., 2004) was fused. Further, Dutch mutant (E22Q) was introduced by PCR using the following primer set. The resultant PCR product was subcloned at a BglII site of pUAST vector. A transgenic *Drosophila* UAS-Dut40 was obtained in the same manner as above.

### (iv) Dut40 (mutation introduction sites were underlined)

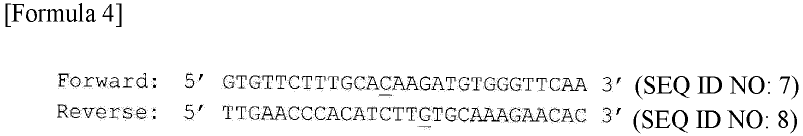

Each of the UAS strains obtained above was crossed with GAL4 strain to prepare *Drosophila* strains expressing the above transgenes. The elav^{C155}-gal4 strain (obtained from Bloomington *Drosophila* stock center) was used as a *Drosophila* strain expressing GAL4, specifically in neurons.

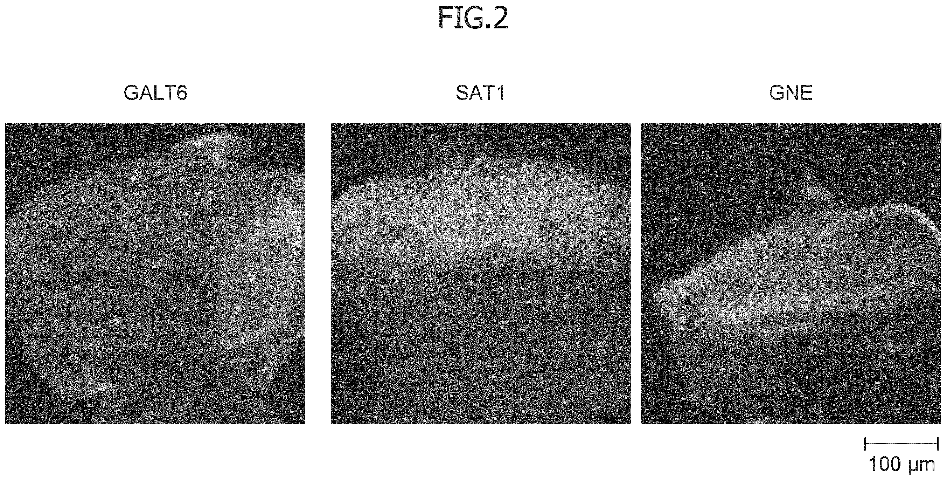

The *Drosophila* expressing the above transgenes were further crossed to prepare a transgenic *Drosophila* expressing GALT6 and SAT1; a transgenic *Drosophila* expressing GALT6, SAT1 and GNE; and a transgenic *Drosophila* expressing GALT6, SAT1 and Dut40. Expressions of GALT6, SAT1 and GNE were checked by immunostaining and the immunostaining results are shown in Fig. 2. Immunostaining was carried out using an anti-GALT6 antibody (20148-1-AP, Proteintech), an anti-SAT1 antibody (ab107534, Abcam) and an anti-GNE antibody (25079-1-AP, Proteintech) in accordance with a routine procedure (Yamasaki et al., Genes Cells., 2011). As a result, it was confirmed that GALT6, SAT1 and GNE were expressed specifically to the neural tissue.

The obtained *Drosophila* strains were grown on Ebios Cornmeal Sucrose Agar medium at a temperature of 25°C, and in a light-dark cycle of 12 hours. In order to provide a sialic acid donor substrate Neu5Ac, *Drosophila* was allowed to freely lick filter paper soaked with a 10 mM Neu5Ac/150 mM sucrose solution for 24 hours every other day for 1 to 2 weeks.

### 2. Production of GM3 ganglioside in transgenic Drosophila

In order to check whether GM3 ganglioside is produced in the transgenic *Drosophila* strains prepared, liquid chromatography mass spectrometry (LC-MS) was carried out. The spectrometry was carried out with respect to the following *Drosophila* strains: (1) wild type (Neu5Ac feeding (-)) (negative control) (2) transgenic *Drosophila* expressing GALT6 alone (Neu5Ac feeding (-)), (3) transgenic *Drosophila* expressing GALT6 and SAT1 (Neu5Ac feeding (-)), (4) transgenic *Drosophila* expressing GALT6 and SAT1 (Neu5Ac feeding (+)), (5) transgenic *Drosophila* expressing SAT1 alone (Neu5Ac feeding (+)), and (6) transgenic *Drosophila* expressing GALT6, SAT1 and GNE (Neu5Ac feeding (-)).

For each of the transgenic *Drosophila* strains, 200 to 1000 heads of adults were collected. To the heads, TBS buffer (50 mM Tris-HCl (pH7.6), 150 mM NaCl) was added, and the mixture was homogenized, and then dried. The obtained pellet was suspended in a solution mixture of chloroform: methanol (v/v = 2: 1) and a lipid extraction liquid was collected. The pellet was further resuspended in a solution mixture of chloroform: methanol: water (v/v/v=2: 1: 0.8) and a lipid extraction liquid was collected. The collected lipid extraction liquid was dried in nitrogen gas at 42°C to obtain a lipid extract. The lipid extract was dissolved in 0.1 N NaOH/methanol, treated at 40°C for 2 hours, neutralized with 0.1 mL of 2N acetic acid, evaporated/concentrated in nitrogen gas, and desalted in C18 BondElut 300 mg cartridge (Agilent Technologies). Thereafter, the lipid extract was dissolved with methanol and used as a sample for LC-MS. LC-MS was carried out by a liquid chromatograph mass spectrometer LCMS-8050 (Shimadzu Corporation).

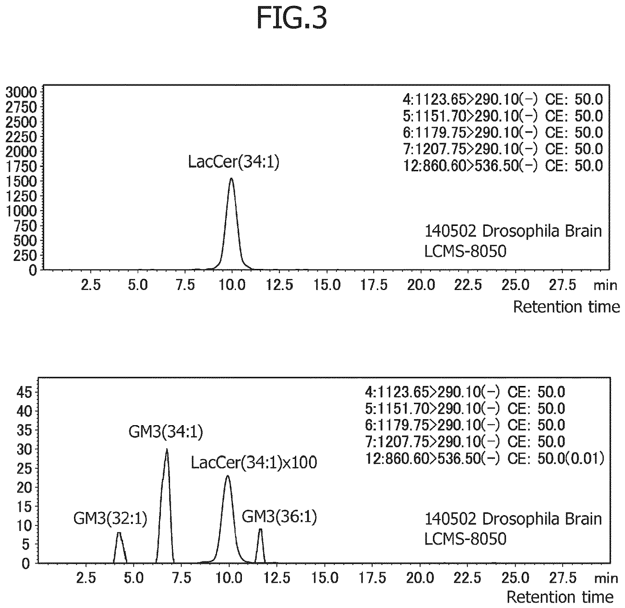

The results are shown in Fig. 3 and Table 1. In the transgenic *Drosophila* expressing GALT6 and SAT1 not fed with a sialic acid donor substrate Neu5Ac, GM3 ganglioside was not produced (Fig. 3 (a)); however, production of GM3 ganglioside was confirmed in the case of *Drosophila* fed with a sialic acid donor substrate Neu5Ac (Fig. 3 (b)). Also, production of GM3 ganglioside was confirmed in the transgenic *Drosophila* expressing GNE in addition to GALT6 and SAT1 (Table 1).

Table 1. Production of LacCer and GM3 in transgenic *Drosophila*

**[Table 1]**

| Genotype | Neu5Ac Feed | LacCer | GM3 |
|---|---|---|---|
| Wild type | - | ND | ND |
| GALT6 | - | + | ND |
| GALT6, SAT1 | - | + | ND |
| GALT6, SAT1 | + | + | + |
| SAT1 | + | ND | ND |
| GALT6, SAT1, GNE | - | + | + |

| | | | |
|---|---|---|---|
| ND: Not detected | | | |

### 3. GM3 ganglioside-dependent Aβ-aggregation in transgenic Drosophila

To confirm whether of GM3 ganglioside-dependent aggregated Aβ is accumulated in the transgenic *Drosophila* co-expressing GM3 ganglioside and Dut40, a sample prepared from the brain tissue of a transgenic *Drosophila* strain expressing GALT6, SAT1 and Dut40, was subjected to western blotting. From 100 heads of adults of each of the transgenic *Drosophila* strain expressing GALT6, SAT1 and Dut40 (Neu5Ac feeding (-)) and the transgenic *Drosophila* expressing GALT6, SAT1 and Dut40 (Neu5Ac feeding (+)), were collected, homogenized in TBS buffer and subjected to ultracentrifugation (100,000 g, one hour, 4°C). The supernatant was collected (TBS soluble fraction). The remaining sediment was again homogenized in 100% formic acid and centrifuged (14,000 rpm, 20 minutes, 25°C) to remove unnecessary components. The supernatant was collected and dried (TBS insoluble fraction). Aβ not aggregated is separated as the TBS soluble fraction and Aβ aggregated and insolubilized is separated as the TBS insoluble fraction.

Samples of individual fractions prepared with Laemmli buffer, and synthesized Aβ-peptide (control), were subjected to 4-20% tris-tricine gel (Cosmo Bio), electrophoresed by SDS-PAGE, and thereafter, transferred to nitrocellulose membrane (NitroBind, MSI). Thereafter, the nitrocellulose membrane was boiled in PSB for 5 minutes and blocked by a 5% skim milk and 0.1% Tween20/PBS solution. Western blotting was carried out using an anti-Aβ monoclonal antibody (clone 82E1, Immuno-Biological Laboratories Co., Ltd.) (1: 100 dilution) as a primary antibody and HRP labeled goat anti-mouse IgG (Cell Signaling Technology Inc.) (1: 3000 dilution) as a secondary antibody. Bands were detected by ECL western blotting detection system (GE Healthcare Life Sciences). The bands detected were analyzed by Image Analysis Software (Imaged, made by the National Institutes of Health) to convert into numerical values. In this manner, the Aβ amounts of individual groups were compared.

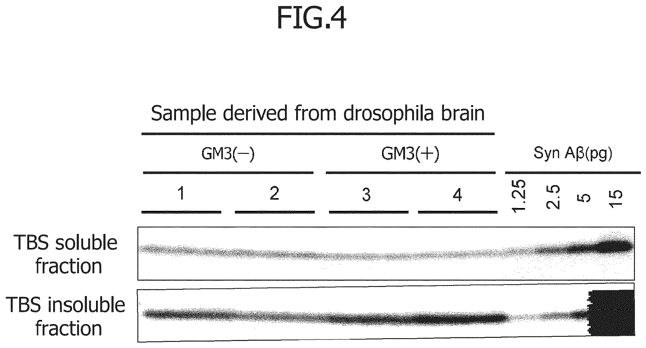

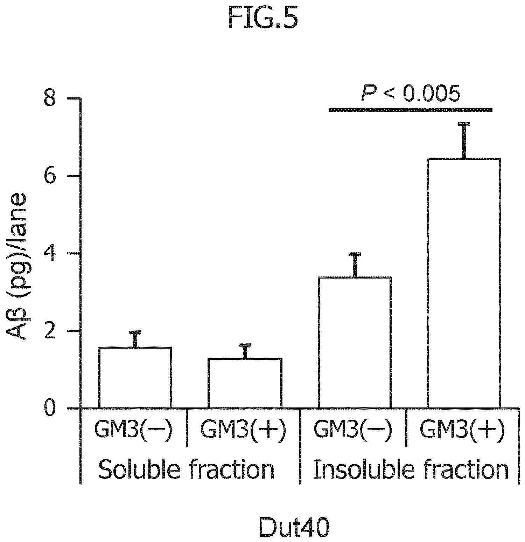

The results are shown in Fig. 4. In samples 3 and 4 (3 lanes for each), which were prepared from the transgenic *Drosophila* strain expressing GALT6, SAT1 and Dut40 (Neu5Ac feeding (+)), the amount of aggregated Dut40 was increased compared to samples 1 and 2 (3 lanes for each), which were prepared from the transgenic *Drosophila* strain expressing GALT6, SAT1 and Dut40 (Neu5Ac feeding (-)). The results of Fig. 4 were converted into numerical values and shown in Fig. 5. From the results, it was demonstrated that GM3 ganglioside-dependent Dut40 aggregation significantly increases.

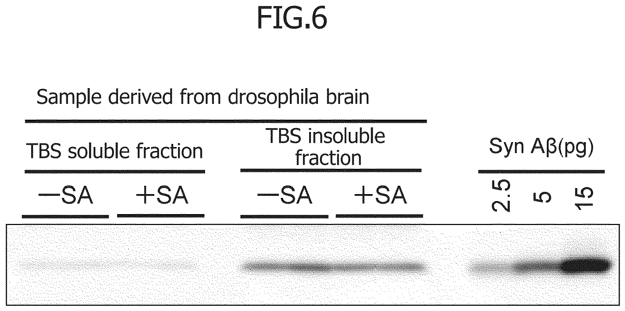

To confirm that Dut40 aggregation is not due to feeding with Neu5Ac, samples were prepared from the transgenic *Drosophila* strains expressing Dut40 (Neu5Ac feeding (-) or (+)) in the same manner as above and subjected to western blotting. The results are shown in Fig. 6. As a result, it was confirmed that feeding of Neu5Ac does not have an effect on the aggregation of Dut40.

The results demonstrated that accumulation of ganglioside-dependent aggregated Aβ in the human brain can be reproduced in a transgenic *Drosophila* co-expressing ganglioside and Aβ.

## Claims

1. A transgenic *Drosophila,* wherein genes encoding mammalian β-1,4-galactose transferase and α-2,3-sialyltransferase are introduced, and GM3 ganglioside is expressed.

2. The transgenic *Drosophila* according to claim 1, wherein a gene encoding an enzyme involved in synthesis of sialic acid is further introduced.

3. The transgenic *Drosophila* according to claim 1 or 2, wherein a gene encoding amyloid β is further introduced, and the GM3 ganglioside, and amyloid β are co-expressed, wherein the amyloid β is a Dutch amyloid β mutant.

4. A method for screening a therapeutic agent for Alzheimer's disease, comprising the steps of:
(1) providing the transgenic *Drosophila* according to claim 3;
(2) administering a candidate compound to the transgenic *Drosophila;* and
(3) analyzing the transgenic *Drosophila* after administration of the candidate compound in step (2).

## Patentansprüche

1. Transgene *Drosophila,* wobei Gene, die für β-1,4-Galactosetransferase und α-2,3-Sialyltransferase aus Säugetieren kodieren, eingeführt werden und GM3-Gangliosid exprimiert wird.

2. Transgene *Drosophila* nach Anspruch 1, wobei ferner ein Gen, das für ein Enzym kodiert, das bei der Synthese von Sialinsäure involviert ist, eingeführt wird.

3. Transgene *Drosophila* nach Anspruch 1 oder 2, wobei ferner ein Gen eingeführt wird, das für Amyloid β kodiert, und das GM3-Gangliosid und Amyloid β co-exprimiert werden, wobei das Amyloid β eine niederländische Amyloid β-Mutante ist.

4. Verfahren zum Screening eines therapeutischen Mittels für Alzheimer-Krankheit, umfassend die folgenden Schritte:
(1) Bereitstellen der transgenen *Drosophila* nach Anspruch 3;
(2) Verabreichen einer Kandidatenverbindung an die transgene *Drosophila,* und
(3) Analysieren der transgenen *Drosophila* nach Verabreichung der Kandidatenverbindung in Schritt (2).

## Revendications

1. Drosophile *transgénique,* les gènes qui codent une β-1,4-galactose transférase et une α-2,3-sialyltransferase de mammifère étant introduits, et le ganglioside GM3 étant exprimé.

2. Drosophile *transgénique* selon la revendication 1, un gène qui code une enzyme impliquée dans la synthèse de l'acide sialique étant en outre introduit.

3. Drosophile *transgénique* selon la revendication 1 ou 2, un gène qui code l'amyloïde β étant en outre introduit, et ledit ganglioside GM3 et ladite amyloïde β étant co-exprimés, ledit amyloïde β étant un mutant d'amyloïde β hollandais.

4. Méthode de criblage d'un agent thérapeutique pour la maladie d'Alzheimer, comprenant les étapes de :
(1) fourniture de la drosophile *transgénique* selon la revendication 3 ;
(2) administration d'un composé candidat à la drosophile *transgénique* ; et
(3) analyse de la drosophile *transgénique* après administration dudit composé candidat à l'étape (2).
